(19) Europäisches Patentamt
European Patent Office
Office européen des brevets

(11) EP 2 451 491 B1

# (12) FASCICULE DE BREVET EUROPEEN

(45) Date de publication et mention de la délivrance du brevet:
**19.11.2014 Bulletin 2014/47**

(21) Numéro de dépôt: **10740344.6**

(22) Date de dépôt: **07.07.2010**

(51) Int Cl.:
***C08G 63/91*** $^{(2006.01)}$ ***A61L 27/14*** $^{(2006.01)}$
***A61L 27/34*** $^{(2006.01)}$ ***A61L 29/14*** $^{(2006.01)}$
***A61L 31/14*** $^{(2006.01)}$ ***C08L 101/06*** $^{(2006.01)}$
***C08L 101/12*** $^{(2006.01)}$ *C08L 67/04* $^{(2006.01)}$

(86) Numéro de dépôt international:
**PCT/IB2010/053111**

(87) Numéro de publication internationale:
**WO 2011/004332 (13.01.2011 Gazette 2011/02)**

## (54) POLYMÈRE HYDROPHOBE POUR LA FABRICATION D’APPAREILS MÉDICAUX VISIBLES EN IRM

HYDROPHOBES POLYMER ZUR HERSTELLUNG VON MRI-SICHTBAREN MEDIZINISCHEN VORRICHTUNGEN

HYDROPHOBIC POLYMER FOR PRODUCING MEDICAL DEVICES VISIBLE IN MRI

(84) Etats contractants désignés:
**AL AT BE BG CH CY CZ DE DK EE ES FI FR GB GR HR HU IE IS IT LI LT LU LV MC MK MT NL NO PL PT RO SE SI SK SM TR**

(30) Priorité: **07.07.2009 FR 0954690**

(43) Date de publication de la demande:
**16.05.2012 Bulletin 2012/20**

(73) Titulaires:
- **Centre National de la Recherche Scientifique 75794 Paris Cedex 16 (FR)**
- **Université de Montpellier 2 34095 Montpellier Cedex (FR)**
- **Universite de Montpellier 1 34967 Montpellier Cedex 2 (FR)**
- **Centre Hospitalier Universitaire de Nîmes 30029 Nimes Cedex 9 (FR)**

(72) Inventeurs:
- **BLANQUER, Sébastien F-34120 Pezenas (FR)**
- **COUDANE, Jean F-34970 Lattes (FR)**
- **DE TAYRAC, Renaud F-30900 Nimes (FR)**
- **GARRIC, Xavier F-34070 Montpellier (FR)**
- **LETOUZEY, Vincent F-34070 Montpellier (FR)**
- **GUILLAUME, Olivier F-35133 Javene (FR)**

(74) Mandataire: **Le Coupanec, Pascale A.M.P. Cabinet Nony 3, rue de Penthièvre 75008 Paris (FR)**

(56) Documents cités:
- **ZHANG, GUODONG ET AL: "Micelles Based on Biodegradable Poly(L-glutamic acid)-b-Polylactide with Paramagnetic Gd Ions Chelated to the Shell Layer as a Potential Nanoscale MRI-Visible Delivery System" BIOMACROMOLECULES , 9(1), 36-42 CODEN: BOMAF6; ISSN: 1525-7797, 2008, XP002600870**
- **SIEVING, PAUL F. ET AL: "Preparation and characterization of paramagnetic polychelates and their protein conjugates" BIOCONJUGATE CHEMISTRY , 1(1), 65-71 CODEN: BCCHES; ISSN: 1043-1802, 1990, XP002600871**
- **REBIZAK, RICHARD ET AL: "Polymeric Conjugates of Gd3+-Diethylenetriaminepentaacetic Acid and Dextran. 2. Influence of Spacer Arm Length and Conjugate Molecular Mass on the Paramagnetic Properties and Some Biological Parameters" BIOCONJUGATE CHEMISTRY , 9(1), 94-99 CODEN: BCCHES; ISSN: 1043-1802, 1998, XP002600872**

Il est rappelé que: Dans un délai de neuf mois à compter de la publication de la mention de la délivrance du brevet européen au Bulletin européen des brevets, toute personne peut faire opposition à ce brevet auprès de l'Office européen des brevets, conformément au règlement d'exécution. L'opposition n'est réputée formée qu'après le paiement de la taxe d'opposition. (Art. 99(1) Convention sur le brevet européen).



Printed by Jouve, 75001 PARIS (FR)

## Description

La présente invention se rapporte au domaine des appareils médicaux, notamment implantables, visibles en imagerie par résonance magnétique.

La présente invention se rapporte plus particulièrement à un nouveau polymère, ayant la propriété d'être hydrophobe et insoluble dans les fluides biologiques, utiles pour la fabrication et/ou le revêtement d'appareils médicaux, notamment implantables, visibles de façon temporaire ou durable en imagerie par résonance magnétique.

L'invention concerne en outre un procédé de préparation d'un tel polymère et un procédé de préparation d'un appareil médical, notamment implantable, détectable en imagerie par résonance magnétique comprenant un tel polymère dans sa masse et/ou à titre de revêtement.

La présente invention concerne en dernier lieu l'appareil médical, notamment implantable, en résultant.

L'imagerie par résonance magnétique (IRM) est un outil d'imagerie médicale qui permet de réaliser des images du corps humain grâce à la présence des atomes d'hydrogène.

Afin d'augmenter l'intensité du signal et la qualité des images obtenues, de nombreux agents de contraste sont utilisés, pour la plupart solubles dans les fluides biologiques, permettant une meilleure visualisation ensuite dans l'organisme.

Toutefois, l'IRM ne permet pas de visualiser une grande majorité des prothèses ou dispositifs médicaux à base de polymère implantés dans l'organisme dans le but de pallier certaines pathologies, et par conséquent de suivre le devenir de ceux-ci dans l'organisme.

Or, il existe un besoin de pouvoir suivre le devenir de ces prothèses ou dispositifs médicaux implantés pour évaluer la qualité et la pérennité de la fixation par exemple, l'intégration cellulaire, ainsi qu'éventuellement la dégradation de la prothèse.

On connaît du document WO 03/045457 un appareil médical implantable comprenant un substrat et un polymère hydrogel de revêtement dudit substrat au moins sur une portion de la surface dudit substrat, dans lequel le polymère hydrogel est adapté pour rendre visible ledit appareil médical en imagerie par résonance magnétique, une fois que ce dernier est implanté chez un patient.

Toutefois, le polymère hydrogel est hydrophile de sorte que les espèces détectables emprisonnées audit polymère hydrogel ne sont pas liées de façon durable à l'appareil médical qui de ce fait ne peut être visible dans la durée, une fois qu'il est implanté.

On connaît également du document US 4 822 594 des agents de contraste, utiles en imagerie par résonance magnétique, comprenant des agents chélatants liés à des polysaccharides.

Toutefois, il s'agit de composés hydrophiles. De plus, le greffage entre les agents chélatants et les polysaccharides s'effectue par la formation d'une liaison ester résultant de la réaction d'un groupement -COOH ou carbonyle de l'agent chélatant directement avec un groupement hydroxyle ou -OH du saccharide.

Ainsi, la position du greffage selon la présente invention, comme exposé ci-après, diffère de celle mise en oeuvre dans ce document.

On connaît par ailleurs du document WO 99/60920 des revêtements susceptibles d'émettre des signaux détectables par résonance magnétique par le biais de la présence d'ions métalliques paramagnétiques utiles pour recouvrir des appareils médicaux lors de processus de diagnostic ou de thérapie mettant en oeuvre l'imagerie par résonance magnétique, telle que la thérapie endo-vasculaire.

Toutefois, la méthode décrite présente des inconvénients majeurs, notamment sur le plan de l'industrialisation en ce qu'elle met en oeuvre en premier lieu une étape dans un réacteur plasma pour prétraiter la surface. Comme cela est exemplifié dans ce document, des surfaces de polyéthylène sont préalablement traitées au plasma à base d'hydrazine, donnant des groupements $NH_2$ libres, préalablement au greffage de l'agent complexant comprenant le métal paramagnétique.

Par ailleurs, cette méthode présente également l'inconvénient de rendre la détermination de la quantité d'ions paramagnétiques délicate car le traitement au plasma ne permet pas d'évaluer précisément la quantité de groupements $NH_2$ libres susceptibles de réagir pour être liés à l'agent complexant.

Il existe par conséquent un besoin de trouver des polymères hydrophobes visibles par imagerie par résonance magnétique susceptibles d'être mis en oeuvre soit en masse soit en surface d'appareils médicaux implantables, la technique associée de préparation desdits polymères étant simple, facile à réaliser et/ou permettant une maîtrise de la quantité d'ions paramagnétiques détectables sur lesdits appareils médicaux.

Selon un premier de ses aspects, la présente invention concerne un polymère hydrophobe, notamment utile pour la fabrication et/ou le revêtement d'appareils médicaux, notamment implantables, visibles en imagerie par résonance magnétique, plus particulièrement de façon durable, caractérisé en ce qu'il comprend au moins une unité monomère sur laquelle est greffé un ligand chélatant d'un ion paramagnétique complexé avec un tel ion paramagnétique, ladite unité monomère possédant au moins un groupe carbonyle, ladite unité monomère comprenant avant greffage au moins un atome d'hydrogène en $\alpha$ dudit au moins un groupe carbonyle et ledit greffage du ligand chélatant s'opérant au niveau

dudit au moins un atome d'hydrogène en α dudit au moins un groupe carbonyle.

Selon un autre de ses aspects, la présente invention concerne un polymère hydrophobe, notamment utile pour la fabrication et/ou le revêtement d'appareils médicaux, notamment implantables, visibles en imagerie par résonance magnétique, plus particulièrement de façon durable, caractérisé en ce qu'il comprend au moins une unité monomère sur laquelle est greffé un ligand chélatant d'un ion paramagnétique complexé avec un tel ion paramagnétique, ladite unité monomère possédant au moins un groupe carbonyle et ladite unité monomère comprenant avant greffage au moins un atome d'hydrogène en α dudit au moins un groupe carbonyle.

Elle a également pour objet un procédé de préparation dudit polymère tel que défini ci-dessus, comprenant (i) au moins une étape d'activation d'un polymère hydrophobe comprenant au moins une unité monomère possédant au moins un groupe carbonyle et comprenant au moins un atome d'hydrogène en α dudit au moins un groupe carbonyle pour former une chaîne polymérique comportant au moins une unité monomère portant un carbanion par l'élimination du proton situé sur le carbone en α dudit au moins un groupe carbonyle, (ii) au moins une étape de greffage sur ledit polymère comportant au moins une unité monomère portant un carbanion avec un ligand chélatant d'un ion paramagnétique, et (iii) au moins une étape de complexation de l'ion paramagnétique avec l'agent chélatant, par exemple par solubilisation du polymère obtenu à l'étape précédente dans un solvant comprenant au moins un ion paramagnétique.

Elle a aussi pour objet un dispositif médical caractérisé en ce qu'il comprend au moins un polymère tel que défini précédemment dans sa masse et/ou à titre de revêtement et/ou à titre de marquage, notamment à vocation de traçabilité comme exposé ci-après.

L'invention a également pour objet un procédé de préparation d'un dispositif médical, notamment implantable, détectable en imagerie par résonance magnétique, caractérisé en ce qu'il comprend au moins une étape de revêtement par un polymère tel que défini ci-dessus, notamment par trempage ou par pulvérisation, dans une solution comprenant ledit polymère conforme à l'invention.

Dans le cadre de la présente invention, on entend par :

- comprendre le polymère « dans sa masse », le fait que l'objet concerné comprenne en son sein un tel polymère, et par exemple soit essentiellement ou partiellement constitué dudit polymère,
- le terme « chaîne polymérique » désigne une macromolécule ou une partie d'une macromolécule comportant une séquence linéaire ou ramifiée d'unités consécutives située entre deux unités consécutives limites qui peuvent être chacune un groupe terminal, un point de branchement ou une particularité caractéristique de la macromolécule,
- le terme « chaîne principale polymérique » désigne la partie linéaire de la chaîne polymérique telle que définie ci-dessus,
- le terme « monomère » couvre une molécule capable d'être convertie en un polymère par combinaison avec lui-même ou avec d'autres molécules du même type,
- une « unité monomère » ou « unité monomérique », désigne la plus petite unité constitutive dont la répétition conduit à une macromolécule régulière,
- un matériau « dégradable hydrolytiquement », un matériau qui se dégrade en présence d'eau consécutivement à la cassure de la liaison ester par hydrolyse et pour lequel on a la preuve que les produits de dégradation du matériau présentent des masses molaires moyennes en nombre inférieures aux masses molaires moyennes en nombre des chaînes polymériques du matériau de départ.
- un matériau « bio-résorbable » ou « résorbable », un matériau qui se dégrade enzymatiquement ou hydrolytiquement et pour lequel on a la preuve que les produits de dégradation sont intégrés en biomasse et/ou éliminés de l'organisme par métabolisation ou filtration rénale,
- «bloc », une partie d'une macromolécule comprenant plusieurs unités constitutives identiques ou différentes et qui possèdent au moins une particularité de constitution ou de configuration permettant de la distinguer de ses parties adjacentes,
- les termes « agent complexant d'un ion paramagnétique », « agent chélatant d'un ion paramagnétique » ou encore « ligand chélatant d'un ion paramagnétique » sont équivalents,
- les termes « compris entre ... et ... » et « varier de ... à ... » signifient que les bornes sont également décrites,
- par polymère hydrophobe, on entend un polymère pour lequel on mesure un angle de contact compris entre 40 et 180° et plus préférentiellement entre 50 et 150°, par exemple selon le protocole de mesure détaillé ci-dessous.

Autrement dit, les polymères conformes à l'invention sont insolubles dans les fluides biologiques.

**<u>Protocole de mesure d'un angle de contact</u>**

La mesure d'angle de contact peut se faire avec un tensiomètre, par exemple KRUSS K100 vendu par la société KRUSS.

D'après la méthode de Wihelmy, on plonge une lame en verre de microscope propre et sèche, dans une solution

de polymère substitué, de concentration 5 g/L dissous dans du tétrahydrofurane (THF), correspondant à une immersion de 1 cm dans l'eau, cette opération étant effectuée à 20 °C.

Le tensiomètre mesure la tension de surface entre l'eau et la lame recouverte de polymère, et calcule l'angle de contact avec l'eau qui en résulte.

## NOUVEAUX POLYMERES HYDROPHOBES

Le polymère hydrophobe selon la présente invention est caractérisé en ce qu'il comprend au moins une unité monomère sur laquelle est greffé un ligand chélatant d'un ion paramagnétique complexé avec un tel ion paramagnétique, ladite unité monomère possédant au moins un groupe carbonyle, et notamment 1 à 3 et ladite unité monomère comprenant avant greffage au moins un atome d'hydrogène en $\alpha$ dudit au moins un groupe carbonyle.

L'invention vise des polymères hydrophobes tels que définis précédemment pouvant présenter des profils de dégradation variés. Autrement dit, suivant la nature du polymère envisagé, comme cela est détaillé ci-après, il peut être biorésorbable ou dégradable hydrolytiquement ou non. Cette propriété peut être ainsi aisément modulée suivant l'application envisagée, ce qui constitue un des avantages de la présente invention. Avantageusement, cette propriété peut être évaluée par mise en oeuvre du test de dégradation suivant.

## Test de dégradation

Ce test permet de déterminer si un polymère est biorésorbable ou dégradable hydrolytiquement conformément à la définition donnée ci-dessus. Ce test consiste à étudier l'évolution, par exemple par chromatographie par exclusion stérique, des masses molaires moyennes en nombre dans des conditions mimant une situation physiologique (tampon PBS à pH 7,4, agitation mécanique à 37 °C).

Le pourcentage de diminution de la masse molaire moyenne en nombre à différents temps est exprimé par l'équation ci-dessous :

$$\% = \frac{\textit{masse molaire moyenne en nombre à } T_0 - \textit{masse molaire moyenne en nombre à } T}{\textit{masse molaire moyenne en nombre à } T_0}$$

A titre indicatif, selon la présente invention :

- pour un temps T = 2 mois, le pourcentage de diminution de la masse molaire moyenne en nombre peut être compris entre 0 et 50 %, de préférence entre 0 et 25 %.
- pour un temps T = 6 mois, le pourcentage de diminution de la masse molaire moyenne en nombre peut être compris entre 0 et 75 %, de préférence entre 5 et 50 %.
- pour un temps T = 1 an, le pourcentage de diminution de la masse molaire moyenne en nombre peut être compris entre 5 et 100 %, de préférence entre 10 et 80 %.
- pour un temps T = 2 ans, le pourcentage de diminution de la masse molaire moyenne en nombre peut être compris entre 10 et 100 %, de préférence entre 20 et 90 %.

Une illustration de mise en oeuvre du test est donnée dans les exemples ci-après au regard d'un polymère conforme à la présente invention.

## POLYMERE DE DEPART

On décrit ci-après les chaînes polymériques pouvant donner naissance aux polymères conformes à la présente invention.

Selon une variante de l'invention, l'unité monomère possédant au moins un groupe carbonyle et comprenant au moins un atome d'hydrogène en $\alpha$ dudit groupe carbonyle, comprend de 2 à 12 atomes de carbone, en particulier de 2 à 7 atomes de carbone.

Selon une autre variante, l'unité monomère possède un groupe carbonyle unique.

Selon un premier mode de réalisation, le groupe carbonyle se situe en dehors de la chaîne principale polymérique. A titre d'exemple d'un tel polymère, on cite notamment les polyacrylates, consistant en totalité ou en partie en des unités monomériques identiques ou différentes, chacun de ces unités présentant la formule (I) suivante :

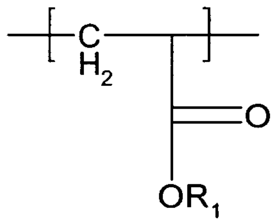

(I)

dans laquelle $R_1$ représente un groupe $(C_1-C_{12})$alkyle ou un groupe $(C_1-C_8)$ cycloalkyle éventuellement substitué par un groupe $(C_1-C_4)$alkyle.

Les polyacrylates sont formés à partir de monomères acrylates.

Parmi de tels monomères acrylates, on peut en particulier citer le butylacrylate, le 2-éthyl-hexylacrylate, le méthylacrylate et l'éthylacrylate.

De tels polymères possèdent la propriété d'être considérés comme non-résorbables et non dégradables hydrolytiquement. Autrement dit, ils ont la faculté de demeurer intact dans le corps humain et de ne pas se dégrader au contact du milieu biologique.

De façon générale, les polyacrylates de formule (I) peuvent être obtenus par polymérisation de monomères acrylates définis ci-dessus.

Selon un deuxième mode de réalisation, le groupe carbonyle se trouve dans la chaîne principale polymérique. A titre d'exemple de ce mode de réalisation, on cite en particulier les polymères polyesters consistant en totalité ou en partie en des unités monomériques identiques ou différentes, chacun de ces unités présentant la formule (II) suivante :

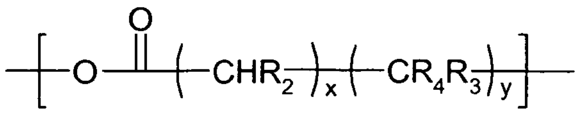

(II)

dans laquelle :

$R_2$, $R_3$ et $R_4$ représentent indépendamment un atome d'hydrogène, un groupe $(C_1-C_{12})$alkyle ou un groupe $(C_1-C_8)$cycloalkyle éventuellement substitué par un groupe $(C_1-C_{12})$alkyle,
x représente un nombre entier compris entre 0 et 12, par exemple entre 0 et 6, et
y représente un nombre entier compris entre 0 et 8, par exemple entre 0 et 6, étant entendu que x et y ne sont pas nuls simultanément.

De façon générale, les polyesters de formule (II) peuvent être obtenus :

a) par polycondensation d'un hydroxyacide sur lui-même, ou
b) par polymérisation par ouverture de cycle lactone.

Parmi ces polymères comprenant des unités monomériques de formule (II), on cite notamment les polyesters consistant en totalité ou en partie en des unités monomériques identiques ou différentes, chacune des unités présentant la formule (III) suivante

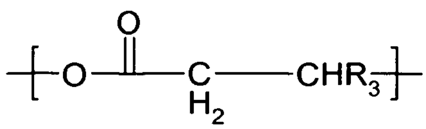

(III)

dans laquelle $R_3$ représente un groupe $(C_1-C_{12})$alkyle.

Parmi les monomères utiles pour préparer des polyesters, on cite notamment l'acide hydroxybutyrique, l'acide hydroxyvalérique, l'acide hydroxyhexanoique et l'acide hydroxyoctanoique.

Le tableau suivant donne la correspondance entre la signification du groupe $R_3$ et le nom complet du polymère de formule (III).

| $R_3$ | Nom du polymère |
|---|---|
| $CH_3$ | Polyhydroxybutyrate (PHB) |
| $C_2H_5$ | Polyhydroxyvalérate (PHV) |
| $C_3H_6$ | Polyhydroxyhexanoate (PHHx) |
| $C_5H_8$ | Polyhydroxyoctanoate (PHO) |

A titre d'illustration d'autres polyesters comprenant des unités monomériques de formule (II), on cite les polyesters obtenus pas ouverture de cycles lactone de formule (IV)

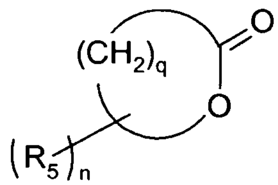

(IV)

dans laquelle :

q représente un nombre entier pouvant varier entre 2 et 9,
$R_5$ représente un groupe ($C_1$-$C_{12}$) alkyle, et
n est un nombre entier compris entre 0 et 2, étant entendu que lorsque n est égal à 2, les deux groupes $R_5$, non seulement peuvent être différents mais encore se situer sur le même ou sur deux atomes de carbone différents.

Lorsque q est égal à 5 et n est égal à 0, il s'agit de la caprolactone ou ε-caprolactone.
Les polyesters ainsi obtenus sont des polycaprolactone ou poly (ε-caprolactone). Parmi les lactones de formule (IV) pouvant convenir à la présente invention, on peut en outre citer la δ-valéro lactone; la γ-butyrolactone; la ε-décatactone, la pivalolactone et la diéthylpropriolactone.
A titre d'illustration d'autres polyesters comprenant des unités monomériques de formule (II), on cite les polymères d'acide lactique (PLA) consistant en totalité ou en partie en des unités monomériques identiques ou différentes, chacune de ces unités présentant la formule suivante :

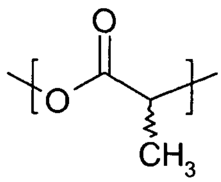

Généralement les polymères d'acide lactique sont obtenus à partir du monomère lactide, par exemple par polymérisation par ouverture de cycle ou à partir d'acide lactique ou de dérivés d'acide lactique par polycondensation. Du fait de la nature chirale de l'acide lactique, il existe le poly-L-lactide (PLLA) et le poly-D-lactide (PDLA), le poly (D,L lactide), le poly-méso-lactide et tous les stéréoisomères qui font partie des polymères conformes pour la présente invention.
A titre d'illustration d'encore d'autres polyesters de formule (II), on cite les polymères d'acide glycolique ou poly(glycolide) consistant en totalité ou en partie en des unités monomériques identiques ou différentes, chacune de ces unités présentant la formule suivante

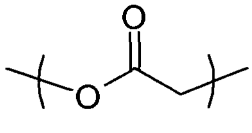

Parmi les polyesters comprenant des unités monomériques de formule (II) utilisables dans le cadre de l'invention, on peut encore citer les homopolymères et copolymères de p-dioxanone (1,4-dioxan-2-one); 1,4-dioxépan-2-one (incluant ses dimères 1,5,8,1 2- tétraoxacyclotétradécane-7, 1 4-dione), 1,4-dioxépan-5-one ; 1,5-dioxépan-2-one; 6,6-diméthyl-1 ; 4-dioxan-2-one; 2,5-dikétomorpholine; 3-methyl-1,4-dioxane-2,5-dione; 3,3-diethyl- 1,4-dioxan-2,5-dione; 6,6-diméthyl- dioxépan-2-one et leurs mélanges de polymères.

La présente invention, selon un de ses aspects, a pour objet un polymère hydrophobe notamment utile pour la fabrication et/ou le revêtement d'appareils médicaux, notamment implantables, visibles en imagerie par résonance magnétique, caractérisé en ce qu'il comprend au moins une unité monomère sur laquelle est greffé un ligand chélatant d'un ion paramagnétique complexé avec un tel ion paramagnétique, ladite unité monomère possédant au moins un groupe carbonyle et ladite unité monomère comprenant avant greffage au moins un atome d'hydrogène en $\alpha$ d'un groupe carbonyle, caractérisé en ce qu'il est issu de la polymérisation d'au moins un monomère choisi parmi l'acide hydroxybutyrique, l'acide hydroxyvalérique, l'acide hydroxyhexanoique et l'acide hydroxyoctanoique ; la $\delta$-valéro lactone ; la $\gamma$-butyrolactone ; la $\varepsilon$-décalactone ; la pivalolactone ; la diéthylpropriolactone ; l'acide glycolique ; la p-dioxanone (1,4-dioxan-2-one) ; la 1,4-dioxépan-2-one ; 1,4-dioxépan-5-one ; la 1,5-dioxépan-2-one; la 6,6-diméthyl-1 ; la 4-dioxan-2-one ; la 2,5-dikétomorpholine ; la 3-methyl- 1,4-dioxane-2,5-dione ; la 3,3-diethyl- 1,4-dioxan-2,5-dione ; la 6,6-diméthyl-dioxépan-2-one et leurs mélanges.

Les polyesters de formule (II) sont considérés comme résorbables ou dégradables hydrolytiquement mais sur le long terme. Ainsi, selon le test de dégradation décrit ci-dessus, les variations de masse molaire moyenne en nombre sont observables dans une période comprise entre 1 semaine et 10 ans.

En réalité, selon la nature du polyester, la résorption peut durer de 1 mois à 10 ans. Dans le cadre de la présente invention, les polyesters ayant un temps de résorption mesuré selon le protocole rappelé ci-dessus supérieur à 1 mois, voire 6 mois, sont plus particulièrement visés.

A titre d'autres polymères possédant un groupe carbonyle dans la chaîne principale polymérique, on peut encore citer les polyethercétones et les polyamidoesters.

Dans le cadre de la présente invention, on entend par :

- un groupe carbonyle un groupe divalent -CO- ;
- (Ct-Cz) où t et z peuvent prendre les valeurs de 2 à 12, un motif carboné pouvant avoir de t à z atomes de carbone, par exemple ($C_2$-$C_3$) un motif carboné qui peut avoir de 2 à 3 atomes de carbone ;
- un groupe alkyle : un groupe aliphatique saturé linéaire ou ramifié. A titre d'exemples, on peut citer les groupes méthyle, éthyle, propyle, isopropyle, butyle, isobutyle, tertbutyle, pentyle, etc ; et
- un groupe cycloalkyle : un groupe alkyle cyclique. A titre d'exemples, on peut citer les groupes cyclopropyle, cyclobutyle, cyclopentyle, cyclohexyle, etc.

Selon un mode de réalisation particulier, on utilise à titre de polymère de départ les homopolymères et copolymères de lactide, glycolide et $\varepsilon$-caprolactone.

Selon un mode de réalisation encore plus particulier, le polymère utilisable dans le cadre de la présente invention est un homopolymère ou un copolymère d'$\varepsilon$-caprolactone, et plus particulièrement un homopolymère d'$\varepsilon$-caprolactone, en particulier de masse molaire moyenne en nombre comprise entre 5 000 et 500 000, en particulier entre 5 000 et 100 000.

Au sens de la présente invention, le polymère hydrophobe conforme à l'invention comprend également, outre les homopolymères, les copolymères (statistiques, blocs, greffés, en peigne ou alternés).

En particulier, l'invention concerne, les polymères di-, tri- ou multiblocs comprenant au moins un bloc comprenant au moins une unité monomère conforme à l'invention, à savoir possédant un groupe carbonyle tel que défini ci-dessus, ainsi qu'au moins un autre bloc pouvant être choisi parmi différents types de blocs biocompatibles, que l'on nommera par la suite par convenance, blocs additionnels.

Par définition, ces blocs additionnels ne comportent pas obligatoirement d'unité monomérique possédant un groupe carbonyle possédant un H en $\alpha$. Parmi ces derniers, on peut citer les blocs polymères suivants :

- poly(éthylèneglycol), poly(propylèneglycol) ou poloxamer.

Selon une variante de l'invention, le bloc additionnel est un bloc poly(éthylèneglycol) ou PEG de formule $H(OCH_2CH_2)_pOH$, où p varie de 10 à 600.

<u>POLYMERE CONFORME A L'INVENTION</u>

Dans le cadre de la présente invention, les polymères conformes à l'invention peuvent présenter une masse molaire moyenne en nombre comprise entre 1 000 et 500 000, notamment entre 5 000 et 100 000 et par exemple entre 10 000 et 50 000.

L'agent complexant utilisé dans le cadre de la présente invention possède au moins une fonction acide carboxylique. A ce titre, on cite notamment l'acide diéthylène triamine penta-acétique (DTPA), l'acide tétraazacyclododacane tétra-acétique (DOTA) et l'acide tétraazacyclotétradécane tétra-acétique (TETA).

L'ion paramagnétique adapté à la présente invention est un métal paramagnétique multivalent incluant, mais non limité aux lanthanides et aux métaux de transition tels que le fer, le manganèse, le chrome, le cobalt et le nickel.

De manière privilégiée, cet ion paramagnétique est un lanthanide qui est hautement paramagnétique et, de manière encore plus préférée, est un ion gadolinium (III).

Selon une première variante, le greffage du ligand chélatant peut être effectué directement sur le polymère.

Selon une deuxième variante, le greffage peut être effectué sur un linker liant le polymère et l'agent chélatant. Parmi les linkers pouvant être utilisés dans le cadre de la présente invention, on peut notamment citer des dérivés contenant au moins deux fonctions susceptibles de réagir avec le carbanion et le chlorure d'acyle de l'agent complexant. On peut citer par exemple, des fonctions alcool, amine ou encore thiol pour réagir sur l'agent complexant et des fonctions halogénures ou halogénure d'acide pour réagir sur le carbanion.

PROCEDE DE GREFFAGE DU LIGAND CHELATANT D'UN ION PARAMAGNETIQUE

Le procédé de préparation consiste de façon générale à fonctionnaliser la chaîne polymérique principale par activation anionique avec une base non nucléophile.

Plus particulièrement, le procédé de préparation du polymère conforme à l'invention peut comprendre (i) au moins une étape d'activation d'un polymère hydrophobe comprenant au moins une unité monomère possédant au moins un groupe carbonyle et comprenant un atome d'hydrogène en $\alpha$ dudit au moins un groupe carbonyle, pour former une chaîne polymérique comportant au moins une unité monomère portant un carbanion par l'élimination du proton situé sur le carbone en $\alpha$ dudit au moins un groupe carbonyle, (ii) au moins une étape de greffage sur ledit polymère comportant au moins une unité monomère portant un carbanion avec un ligand chélatant d'un ion paramagnétique, et (iii) au moins une étape de complexation de l'ion paramagnétique avec l'agent chélatant, par exemple par solubilisation du polymère obtenu à l'étape précédente dans un solvant comprenant au moins un ion paramagnétique.

Le schéma général, en partant des polymères de départ définis ci-dessus, comprenant respectivement des unités monomériques de formule (I) ou (II) peut être représenté selon le schéma 1 suivant :

**Schéma 1**

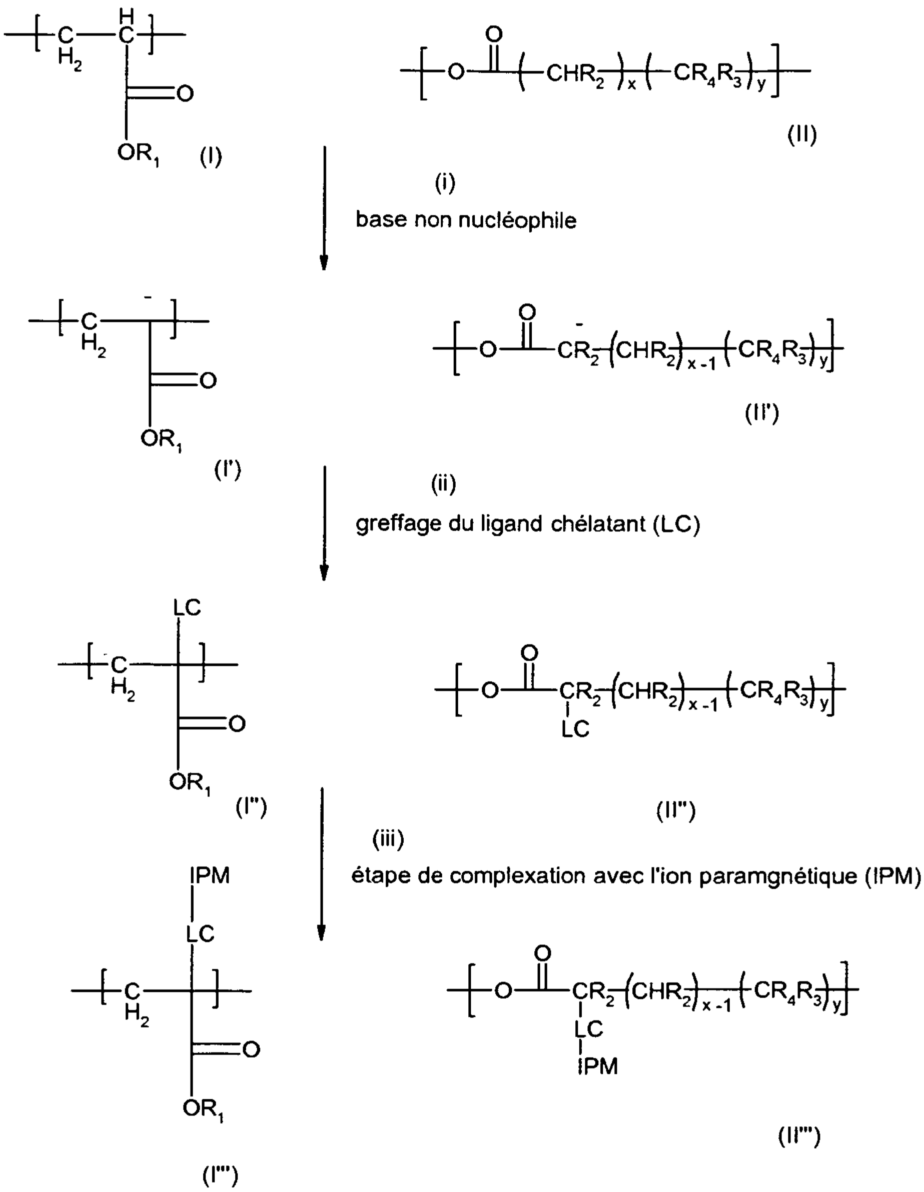


(I) (II)

(I') (II')

(I") (II")

(I"') (II"')

La première étape (i) d'activation peut être obtenue par utilisation d'une base forte telle que le di-isopropylamide de lithium (LDA) par exemple dans un milieu organique anhydre. Des intermédiaires de type carbanions et/ou énolates sont obtenus sur la chaîne principale polymérique. Ces intermédiaires sont nommés carbanions dans la suite de la description, par exemple de formule (I') ou (II') selon le schéma (I) ci-dessus.

Cette première étape (i) peut être mise en oeuvre de la même manière, que la fonction ester se situe sur la chaîne principale polymérique ou sur des chaînes latérales, comme cela ressort de la description ci-dessus.

Typiquement, la première étape (i) de la réaction décrite ci-dessus peut prendre de 5 à 60 minutes, par exemple de 15 à 45 minutes.

La réaction selon cette première étape (i) peut être effectuée à des températures variant de -70 à 30 °C, par exemple de -60 à 0 °C.

Cette première étape (i) de préparation du polymère, conforme à l'invention, peut notamment être effectuée selon les modes opératoires décrits dans l'article «A Novel Route to Poly(ε-caprolactone) Based Copolymers Via Anionic Derivativation », S. Ponsart et al., Biomacromolecules 2000, 1, 275-281.

Il est entendu que le degré ou taux de substitution du polymère par le ligand chélatant dépend fortement de cette 1$^{ère}$ étape (i). En particulier, la quantité de base forte introduite influe sur ce taux de substitution. Toutefois, ce dernier plafonne à une certaine valeur suivant le polymère de départ, même lorsque la base forte est introduite en excès.

De la même façon, la température de réaction de cette première étape (i) influence également ledit taux de

substitution. Plus la température de réaction est élevée, plus le taux de substitution est également élevé, tout en restant inférieur à la valeur plafond évoquée ci-dessus.

D'autres paramètres peuvent également influencer le taux de substitution et notamment la nature de la base et la durée de la réaction.

Selon la deuxième étape (ii), on procède au greffage de l'agent complexant sur le polymère comprenant au moins une unité polymérique sous forme de carbanion.

Cet agent complexant peut avoir été au préalable modifié chimiquement de façon à obtenir un produit soluble dans le même solvant que le polymère et présentant une fonction réactive pour le greffage.

Selon ce procédé, le greffage peut alors être obtenu entre le polymère comprenant au moins l'unité polymérique sous forme de carbanion. Le greffage peut par exemple être effectué dans un solvant tel que le tétrahydrofurane, le toluène, des dérivés aromatiques du benzène, le dioxane et d'une manière générale, tout solvant ne réagissant pas avec la base choisie pour la réaction ou le carbanion à une température comprise entre -70 et 30 °C, par exemple à -40 °C, entre 5 et 60 minutes, par exemple pendant 30 minutes.

Selon la troisième étape (iii), on peut finalement obtenir le polymère conforme à la présente invention par réaction du produit obtenu selon l'étape précédente dans un solvant tel que par exemple le DMSO ou un mélange de solvants par exemple eau/acétone contenant un ion paramagnétique, par exemple le trichlorure de gadolinium, et agitation entre 1 heure et 10 jours, par exemple 2 jours, par exemple à température ambiante.

Le polymère obtenu à l'issu de ce procédé présente la caractéristique d'être hydrophobe et insoluble dans les solvants aqueux, par exemple les fluides biologiques. Ce polymère ainsi obtenu présente sur sa chaîne des substituants (agent complexant + ion paramagnétique) qui le rendent visible en IRM.

D'une façon générale, le taux de substitution de l'agent chélatant varie de 0,01 à 40 %.

Plus particulièrement, le taux de substitution pouvant être obtenu lorsque le polymère comprend au moins un groupe carbonyle dans la chaîne principale polymérique peut aller de 0,01 à 10 %, par exemple être inférieur à 10 %.

De même, le taux de substitution peut être compris entre 0,01 et 40 %, par exemple être inférieur à 30 % lorsque au moins un groupe carbonyle se situe en dehors de la chaîne principale polymérique, autrement dit est compris dans une chaîne latérale à la chaîne polymérique principale.

## DISPOSITIF MEDICAL

La présente invention s'étend à un dispositif médical comprenant au moins un polymère conforme à la présente invention.

Le polymère conforme à la présente invention peut faire partie intégrante du dispositif médical en lui-même ; il est autrement dit compris dans sa masse ou bien se trouver en surface dudit dispositif sous forme d'un revêtement d'une épaisseur telle qu'elle permette de rendre le dispositif médical visible en imagerie par résonance magnétique.

Typiquement, le revêtement peut former une épaisseur comprise entre 1 et 1000 $\mu$m, par exemple entre 10 et 100 $\mu$m.

Dans la variante consistant à revêtir le dispositif médical, différentes méthodes peuvent être employées connues en elles-mêmes par l'homme de l'art. A ce titre, on peut citer l'électro-spinning, le trempage, la mise en oeuvre d'un spray drying ou aérographe ou pulvérisation.

Bien entendu, la présente demande s'étend aux dispositifs médicaux ayant subi un autre type de traitement en masse et/ou de surface d'une autre nature que celle considérée dans la présente invention. A titre d'exemple, on peut citer les traitements antiadhérentiels bactériens et fongiques, les traitements permettant une libération de principes actifs tels que des antibiotiques, des antibactériens, des antifongiques, des antiinflammatoires, et toute sorte de principes actifs pouvant être libérés *in situ.*

A titre de dispositifs médicaux particulièrement adaptés à la présente invention, on peut citer les dispositifs médicaux trouvant plus particulièrement une application dans le domaine de la gynécologie par exemple pour des treillis ou prothèses pour prolapsus génital.

Selon un autre aspect, la présente invention s'étend à un procédé de marquage d'un dispositif médical, caractérisé en ce qu'il comprend au moins une étape de dépôt, notamment sur une zone ciblée du dispositif médical, sur la surface du matériau, d'un polymère conforme à l'invention.

Selon cet aspect, le polymère selon l'invention est ainsi déposé sur les prothèses, notamment sous la forme d'une inscription afin d'effectuer un suivi postopératoire grâce au marquage direct sur le matériau.

Plus particulièrement, le marquage peut être destiné à la traçabilité du dispositif médical. Le dispositif médical peut ainsi être identifié tout au long de sa vie, que ce soit lors de la fabrication, de la distribution ou encore une fois posé. Ainsi le marquage peut prendre toute forme ou surface sur le dispositif médical.

Encore plus particulièrement, les inscriptions peuvent prendre la forme de chiffres, de lettres ou de tout autre type de caractères utiles à la traçabilité.

A titre d'exemples, le marquage peut être effectué selon l'un des procédés suivants :

- le dépôt d'une surface homogène de polymère selon l'invention à la surface du matériau, dans lequel les caractères sont gravés.

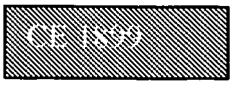


- le dépôt des caractères à la surface du matériau par des techniques de micro-imprimerie, dans lequel le polymère conforme à l'invention représente l'encre.

| CE 1899 |
|---|

Cet aspect de l'invention est particulièrement avantageux dans la mesure où le marquage est ainsi solidaire du dispositif médical et non plus de l'emballage de celui-ci comme c'est habituellement le cas.
L'invention s'étend également à un dispositif médical doté d'un marquage réalisé à l'aide d'un polymère selon la présente invention.
Le polymère selon la présente invention peut également trouver son application dans tout domaine médical où l'IRM est utilisée.
Ces différents domaines peuvent être classés par spécialités chirurgicales ou par type de matériaux notamment implantables qui sont utilisés dans plusieurs domaines médicaux.

<u>Classement par spécialités médico-chirurgicales :</u>

*<u>Gynécologie :</u>*

- treillis de soutènement dans la cure de prolapsus génito urinaire et rectal (chirurgie vaginale et abdominale)
- clips de stérilisation tubaire
- dispositifs d'obstruction tubaire par voie endo-luminale
- bandelette de cerclage du col utérin
- dispositifs anti adhérentiel intra péritonéaux et endo utérins

*<u>Urologie :</u>*

- Sphincter Urinaire Artificielle
- prothèse pénienne
- patch de renfort des corps caverneux (cure de coudure de verge, maladie de Lapeyronie)
- ballonnets péri-urétraux
- dispositifs injectables en péri-urétral
- bandelettes sous urétrales
- stent, prothèses endo-urétrale
- sondes de dérivation urinaire (trans cutanées et voies naturelles).

*Orthopédie :*

- ligaments synthétiques
- néo cartilage ou articulation
- disques inter vertébraux synthétiques
- tête fémorale
- cotyle (fémur et humerus)
- plateau tibiale
- tète humérale.

*ORL :*

- implants cochléaires
- prothèses de l'oreille interne, substituts osseux.

*Endocrinologie :*

- pompes implantables.

*Vasculaire :*

- prothèses endo-vasculaires
- prothèses artérielles et veineuses
- dispositifs de clôture et d'hémostase d'abord vasculaire trans artérielle
- boitiers et cathéters de chambres d'abord vasculaire implantables.

*Neurologue:*

- Stents vasculaires
- dispositifs d'occlusion d'anévrysme et dissection vasculaire artérielle
- sondes d'électrostimulation
- patchs et renforts de dure mère et méningée.

*Ophtalmologie :*

- cornées synthétiques.

*Chirurgie digestive:*

- plaques de renfort (treillis) herniaire (diaphragmatique, pariétale, inguinal, crurale)
- anneaux gastriques
- filets spléniques
- prothèses oesophagiennes
- stents de voie biliaire et digestive (grêle, colon, rectum) endoprothèse
- sondes de nutrition parentérale
- sphincter anale artificielle.

*Cardiologie :*

- stents coronariens
- étuis et sondes de Pace Maker
- sondes d'entrainement systolique

*Radiologie :*

- agents d'embolisation vasculaire, d'occlusion vasculaire (artérielle ou veineux) (temporels ou définitifs.

Matériaux implantables utilisés dans plusieurs domaines médicaux :

- Fils de sutures chirurgicales.
- Cathéters veineux et artérielles (centraux et périphériques).
- Sondes thermiques endo corporelles.
- Drains chirurgicaux, tubulaires et lames, crains de drainage.
- Agrafes synthétiques de rapprochement, de repérage, pour anastomose digestive et de fixation prothétique.
- Ingénierie tissulaire : Matrice de support de cellules souches en chirurgie réparatrice.

Le polymère selon l'invention peut, enfin, trouver une application dans l'enceinte même de l'appareillage IRM.
L'environnement de travail dans la salle de l'IRM rend nécessaire l'absence de métaux capable de perturber

le champ magnétique de l'IRM. Cet environnement implique notamment le développement de matériel de ventilation et de réanimation sans métal, IRM compatible. On peut citer à ce titre le matériel suivant : table, têtière, minerve, attelle, harpons, appuis et cals permettant la mise en position et repérage en IRM.

Les exemples qui suivent illustrent l'invention sans en limiter la portée.

## EXEMPLES

### Exemple 1 : préparation à base d'un polymère pohy ε-canrolactone

La caractérisation des produits est illustrée par les figures en annexe. Le schéma de synthèse est le suivant :

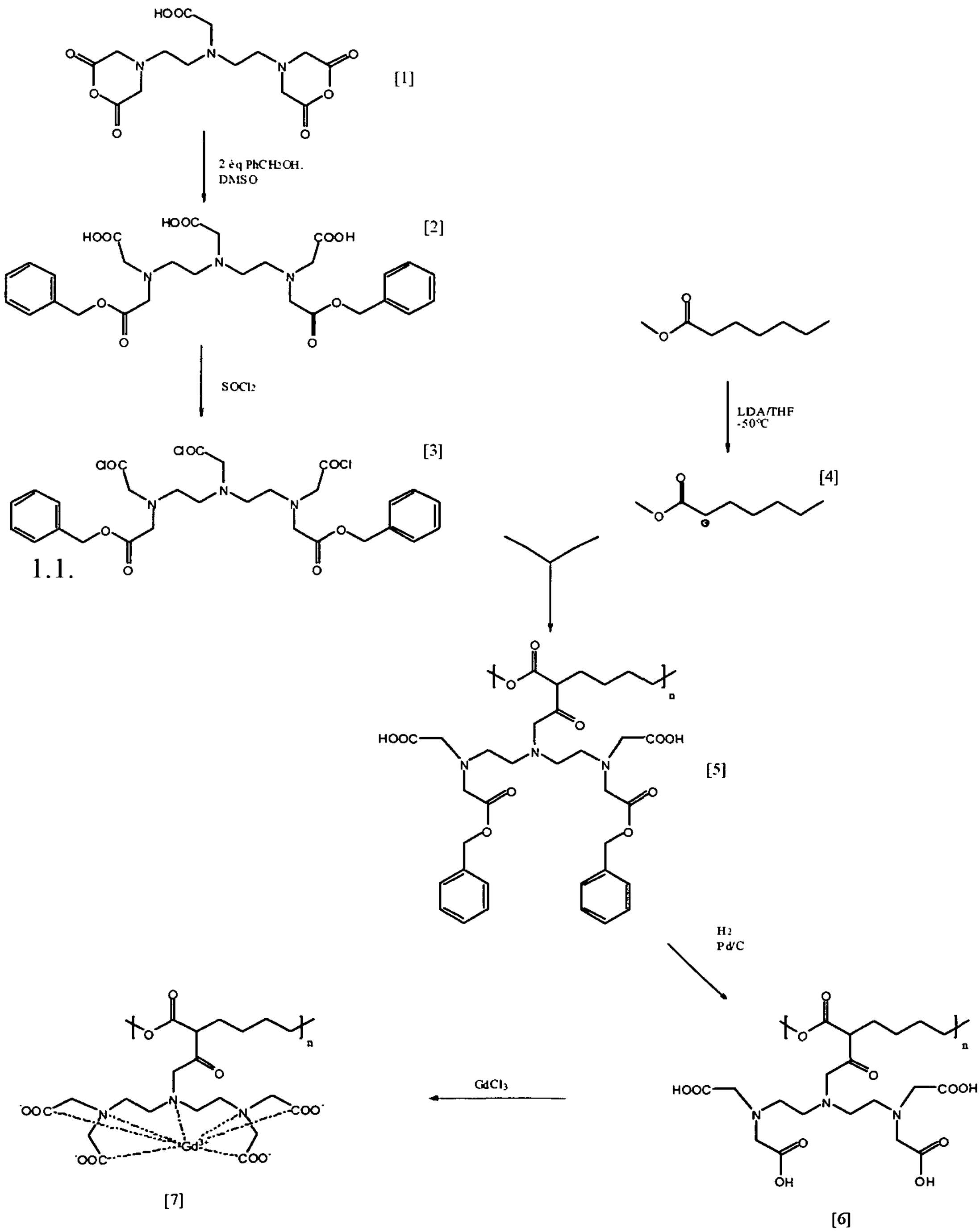

[1] [2] [3] 1.1. [4] [5] [6] [7]

Synthèse du DTPA diphénylé [2] (M = 550 g/mol) :

A une suspension de 1 g de DTPA dianhydride [1] dans le DMSO (insoluble à température ambiante), sont ajoutés 730 µl d'alcool benzylique (2,5 éq.). Le mélange est laissé 5 heures à température ambiante, et l'évolution de la réaction est suivie par spectrométrie Infra-Rouge (disparition du pic caractéristique du dianhydride à 1800 $cm^{-1}$).

Traitement :

On fait évaporer le DMSO et l'alcool benzylique en excès pendant 2 jours à l'aide d'une pompe à palettes. Le produit obtenu est recristallisé dans l'éther éthylique. On récupère une poudre blanche.

Caractérisations :

- HPLC : L'utilisation de l'HPLC en mode isocratique avec comme éluant un mélange $H_2O$/acétonitrile (65/35), la détection se faisant par UV à une longueur d'onde de 257 nm, qui permet de mettre en évidence la présence d'une nouvelle structure (DTPA diphenylé). La figure 1 représente les chromatogrammes obtenus par HPLC en mode isocratique avec un ratio 65/35 $H_2O$/ACN, du DTPA-Ph, alcool benzylique et DTPA.

  Temps de rétention (DTPA diphenylé) = 6,75min
  Temps de rétention (alcool benzylique)= 3,7min
  Temps de rétention (DTPA) = 1,9min

- Spectrométrie Infra Rouge : L'analyse infra-rouge révèle la disparition du pic caractéristique de l'anhydride, à 1800 $cm^{-1}$. La figure 2 représente les spectres Infra-rouge du DTPA dianhydride (A) et DTPA-diphénylé (B).
- Résonance Magnétique Nucléaire 1H (DMSO D6) : La RMN du proton renseigne sur la structure du produit et sur la présence de deux groupements aromatiques sur le DTPA.

2,9 ppm (m, 8H, H1), 3,4 ppm (m, 6H, H2), 3,6 ppm (m, 4H, H4) 5,1 ppm (m, 2H, H5), 7,3 ppm (m, 10H, aromatique H6)

En comparaison avec l'alcool benzylique:

Alcool : 4,3ppm (d, CH2); 5,2ppm (t, OH); 7,15ppm (aromatique)

1.2. Synthèse du DTPA-diPH-Cl [3]:

1 g de DTPA diphénylé est dissous dans 8 ml de $SOCl_2$. Le mélange est laissé à température ambiante 2 heures, une huile brun foncé est récupérée après évaporation du $SOCl_2$ en excès, puis solubilisée dans le THF. Le produit brut est utilisé directement dans l'étape suivante.

1.3. Synthèse de la PCL-DTPA-diPh[5] :

2 g de PCL sont dissous dans 200 ml de THF à -40 °C, 17,5 ml d'une solution 2N de LDA (Lithium Diisopropylamine) (2 éq.) sont ajoutés goutte à goutte sous agitation. La réaction est poursuivie 30 minutes à -40 °C et permet d'obtenir le carbanion de la PCL[4]. Le DTPA-diPH-Cl est dilué dans 20ml de THF puis ajouté goutte à goutte au carbanion en 30 minutes, à -40 °C.

Traitements :

On procède à la neutralisation par 150 ml de $H_2O$/HCl jusqu'à pH 4-5, puis à une extraction liquide 3 fois par le dichlorométhane, suivie d'un lavage à l'eau de la phase organique et enfin d'une décantation, récupération de la phase organique qui est évaporée. Le produit obtenu est précipité dans le MeOH puis filtré.

Caractérisations :

- Chromatographie d'exclusion stérique (CES) : Le détecteur utilisé pour la CES est un détecteur UV-Visible à barrettes

de diodes (PDA). La CES montre la présence d'un polymère absorbant à la longueur d'onde de 292 nm. La PCL n'absorbant pas à cette longueur d'onde, ce nouveau produit correspond à de la PCL modifiée qui correspond à de la PCL-DTPA diphenylé. La figure 3 représente le chromatogramme à $\lambda$=292 nm de la PCL- DTPA-diPh obtenu par Chromatographie d'Exclusion Stérique couplé à une détecteur à barrettes de diodes..

- RMN 1H : La RMN du proton met en évidence la substitution et permet de mesurer le taux de greffage du DTPA sur la PCL. La comparaison des rapports d'intensité des intégrations des protons aromatiques et des protons du motif caprolactone de la PCL, donne le taux de substitution qui est voisin de 2 %.
  4,0ppm (t, $CH_2$-O); 2,2ppm (t, $CH_2$-CO-O); 1,5ppm (m, 2 $CH_2$ en gamma et epsilon); 1,3ppm (m, $CH_2$ centre); 5,1ppm ($CH_2$ aromatique).
- Dosage acido-basique : Il consiste à doser les fonctions acide carboxylique présentes sur le DTPA greffé et permet donc de déterminer le taux de substitution sur la PCL.

Ce dosage nous indique que la PCL a été substituée à 2,5 % par le DTPA diphénylé.

1.4. Synthèse de la PCL-DTPA [6]

Cette étape consiste à déprotéger par débenzylation le DTPA greffé sur le polymère.
PCL-DTPA-Ph (1 g) est dissous dans 80 ml de THF. 50 mg d'une suspension de Pd/C à 10 % sont ajoutés à la solution. Le mélange est laissé sous une pression de 4 bars d'$H_2$ pendant 3 jours à TA.

<u>Traitement :</u>

Après filtration puis évaporation du THF, le polymère est reprécipité dans le méthanol.

<u>Caractérisations :</u>

CES avec détecteur PDA: Le chromatogramme du polymère obtenu montre que le polymère ne contient plus d'aromatiques et que l'alcool benzylique a été éliminé.
RMN$^1$H : Sur le spectre RMN, on observe, par rapport à la PCL-DTPA-diphénylée, la disparition des pics correspondant aux protons aromatiques.

1.5. Synthèse de la PCL-DTPA-Gd [7]

100 mg du polymère modifié sont dissous dans du DMSO, et mélangés à 30 mg d'une solution de $GdCl_3$ (10 éq. par rapport au DTPA greffé) dans 10 ml de DMSO. Le mélange est mis sous agitation à température ambiante pendant deux jours pour achever la complexation.

<u>Traitement :</u>

Evaporation du DMSO, et extraction $CH_2Cl_2/H_2O$. Evaporation du $CH_2Cl_2$ et reprécipitation dans le MeOH.

<u>Caractérisation :</u>

- Temps de relaxation des protons de la PCL:
Les résultats ci-après correspondent aux temps de relaxation des protons de la chaîne PCL, substituée ou non. Ils montrent la présence de l'ion $Gd^{3+}$ dans PCL-DTPA-Gd

| déplacement chimique | temps de relaxation (ms) | |
|---|---|---|
| | PCL | PCL-DTPA-Gd |
| 4,00 ppm | 693,43 | 480,78 |
| 2,28 | 693,06 | 525,93 |
| 1,54 | 632,62 | 456,00 |
| 1,31 | 671,10 | 475,15 |

- IRM : Après analyse des images annexées en figure 4, le puits N°3 correspondant à une PCL-DTPA-Gd substituée à 2 % donne un signal positif significatif. En comparaison le puits N°12 correspondant à de la PCL mélangée avec du

Magnevist (produit de contraste commercial d'IRM), donne également un signal positif significatif. La figure 4 représente les images IRM des différents polymères greffés et des témoins négatifs et positifs IRM (3 Tesla). Toujours sur cette figure 4 sont représentés différents polymères greffés avec différents taux de substitution et différentes méthodes de complexation (Figure 4-A; Séquence 2D pondérée T2) témoins négatifs (8-11) et positifs (12) (Figure 4-B ; Séquence 2D pondérée T1).

**Exemple 2 :**

*Méthode de pulvérisation, pour obtenir un revêtement visible en IRM.*

Afin de fournir un caractère de visibilité en IRM à un dispositif médical de type treillis, une méthode de pulvérisation à l'aide d'un aérographe est développée pour cette application (aérographe Infinity® de la marque Harder & Steenbeck muni d'une buse de 0,15 mm et d'un godet de 5 mL).

Pour cela, il est nécessaire de dissoudre le polymère dans un solvant organique facilement volatilisable (de type acétone, chloroforme, dichlorométhane...). La concentration en polymère dans le solvant doit être comprise entre 1 et 5 % (masse par volume) afin d'obtenir une solution limpide facilement pulvérisable et de manière homogène et continue.

La solution de polymère est pulvérisée sous pression à 2,5 bars à une distance de 5 cm de treillis directement sur sa surface, soit de manière localisée soit sur la totalité de la surface. Un dépôt de 20 à 50 $\mu$m de polymère à la surface de l'implant est nécessaire pour permettre une visualisation IRM de celui-ci, tout en conservant les propriétés intrinsèques de l'implant.

Un séchage du treillis pendant 12 heures sous vide est réalisé pour évaporer la totalité du solvant. La quantité de polymère déposé sur l'implant est déterminée en comparant le poids de l'implant avant et après pulvérisation.

***Angle de contact***

L'angle de contact est mesuré selon le protocole décrit précédemment.

- Lame seule : 31 °
- Lame contenant PCL-DTPA-Gd : 80°
- Lame contenant PCL : 82°

On vérifie bien par ce test que le polymère obtenu est hydrophobe.

***Test de dégradation***

On dépose dans 30 tubes une quantité définie de polymère substitué, par exemple 50 mg pour chaque tube, dans lequel on ajoute 5 ml de PBS. Ces tubes sont ensuite mis dans une étuve à 37 °C sous agitation pour mimer des conditions physiologiques.

3 tubes correspondent à un temps de dégradation :

- 7 jours - 150 jours
- 15 jours - 210 jours
- 30 jours - 270 jours
- 60 jours - 330 jours
- 90 jours - 400 jours

Chaque échantillon est ensuite filtré et analysé par Chromatographie par Exclusion Stérique. On compare les masses moyennes en nombre et les masses molaires au sommet du pic de chaque échantillon pour suivre la dégradation par rapport aux masses molaires initiales du polymère de départ.

La figure 5 représente l'évolution des masses molaires moyennes en nombre (Mn) et au somment du pic (Mp) pendant 210 jours. Quel que soit le type de masse molaire, une diminution notable des masses molaires est observable (environ 20% en 210 jours).

***<u>Visualisation du treillis in vitro</u>***

On visualise le treillis par un appareillage : Imageur 7T Bruker DRX300SWB, configuration « mini-imagerie » (gradient 144mT/m, résonateur « cage d'oiseau » 64mm).

La figure 6 représente (i) le treillis en témoin négatif pour ce qui est des deux images de gauche, à savoir un treillis enduit avec la PCL-DTPA sans Gadolinium et (ii) l'échantillon, enduit avec le PCL-DTPA-Gd tel que préparé à l'exemple 1 pour ce qui est des deux images de droite.

Par ailleurs, cette figure 6 se comprend comme suit :

a/ Treillis comportant un revêtement vue au microscope optique (grossissement X 20).

b/ Analyse IRM en échos de spin 2D avec motif d'inversion, donc séquence pondérée T2 sensibilisée T1.

***<u>Visualisation du treillis in vivo</u>***

Un rat a subi une implantation dorsale de 4 prothèses (1 x 3 cm) type « mesh » (2 recouvertes de polymères visibles en IRM et 2 recouvertes de polymères non visibles en IRM). Les implantations ont été effectuées en sous-cutanée et en intra-musculaire. On visualise le treillis par un appareillage : Imageur 7T Bruker DRX300SWB, configuration « mini-imagerie » (gradient 144mT/m, résonateur « antennes de surface » 1H/31P). La figure 7 représente le rat en séquence d'écho de gradient 3D (A) et en séquence d'écho de gradient 3D de type FLASH avec angles de basculement variables (VFA= variable flip angle) (B).

Ces photographies démontrent que les treillis sont bien visualisables 3 semaines après l'implantation.

## Revendications

1. Polymère hydrophobe, notamment utile pour la fabrication et/ou le revêtement d'appareils médicaux, notamment implantables, visibles en imagerie par résonance magnétique, **caractérisé en ce qu'**il comprend au moins une unité monomère sur laquelle est greffé un ligand chélatant d'un ion paramagnétique complexé avec un tel ion paramagnétique, ladite unité monomère possédant au moins un groupe carbonyle, ladite unité monomère comprenant avant greffage au moins un atome d'hydrogène en $\alpha$ dudit au moins un groupe carbonyle et ledit greffage du ligand chélatant s'opérant au niveau dudit au moins un atome d'hydrogène en $\alpha$ dudit au moins un groupe carbonyle.

2. Polymère selon la revendication précédente, **caractérisé en ce que** l'unité monomère possédant au moins un groupe carbonyle et comprenant avant greffage un atome d'hydrogène en $\alpha$ dudit au moins un groupe carbonyle, comprend de 2 à 12 atomes de carbone, en particulier de 2 à 7 atomes de carbone.

3. Polymère selon l'une des revendications 1 ou 2, **caractérisée en ce que** l'unité monomère possédant au moins un groupe carbonyle et comprenant avant greffage au moins un atome d'hydrogène en $\alpha$ dudit au moins un groupe carbonyle se situe en dehors de la chaîne principale polymérique ou alternativement ledit au moins un groupe carbonyle se trouve dans la chaîne principale polymérique.

4. Polymère selon l'une quelconque des revendications précédentes, **caractérisé en ce qu'**il s'agit d'un polyacrylate consistant en totalité ou en partie en des unités monomériques identiques ou différentes, chacune de ces unités possédant la formule (I) suivante

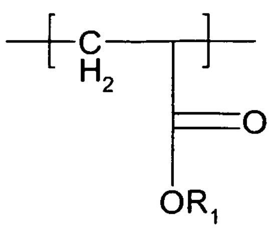
(I)

dans laquelle $R_1$ représente un groupe $(C_1-C_{12})$alkyle ou un groupe $(C_1-C_8)$cycloalkyle éventuellement substitué par un groupe $(C_1-C_4)$alkyle, ladite unité monomérique pouvant notamment être issue de monomères choisis parmi le butylacrylate, le 2-éthyl-hexylacrylate, le méthylacrylate et l'éthylacrylate.

5. Polymère selon l'une quelconque des revendications précédentes, **caractérisé en ce qu'**il est issu d'un polyester consistant en totalité ou en partie en des unités monomériques identiques ou différentes, chacune de ces unités présentant la formule (II) suivante :

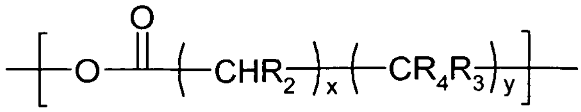

**(II)**

dans laquelle :

$R_2$, $R_3$ et $R_4$ représentent indépendamment un atome d'hydrogène, un groupe ($C_1$-$C_{12}$) alkyle ou un groupe ($C_1$-$C_8$) cycloalkyle éventuellement substitué par un groupe ($C_1$-$C_{12}$) alkyle,
x représente un nombre entier compris entre 0 et 12, par exemple entre 0 et 6, et
y représente un nombre entier compris entre 0 et 8, par exemple entre 0 et 6, étant entendu que x et y ne sont pas nuls simultanément.

**6.** Polymère selon la revendication précédente, **caractérisé en ce qu'**il est issu de la polymérisation d'au moins un monomère choisi parmi l'acide hydroxybutyrique, l'acide hydroxyvalérique, l'acide hydroxyhexanoique et l'acide hydroxyoctanoique ; la δ-valéro lactone ; la γ-butyrolactone ; la ε-décalactone ; la pivalolactone ; la diéthylpropriolactone ; l'acide glycolique ; la p-dioxanone (1,4-dioxan-2-one) ; la 1,4-dioxépan-2-one ; 1,4-dioxépan-5-one ; la 1,5-dioxépan-2-one; la 6,6-diméthyl-1 ; la 4-dioxan-2-one ; la 2,5-dikétomorpholine ; la 3-methyl- 1,4-dioxane-2,5-dione ; la 3,3-diethyl- 1,4-dioxan-2,5-dione ; la 6,6-diméthyl-dioxépan-2-one et leurs mélanges.

**7.** Polymère selon l'une quelconque des revendications précédentes, **caractérisé en ce qu'**il est issu de poly(ε-caprolactone).

**8.** Polymère selon l'une quelconque des revendications précédentes, **caractérisé en ce qu'**il s'agit d'un homopolymère, ou d'un copolymère, notamment statistique ou en bloc, greffé, en peigne ou alterné.

**9.** Polymère selon l'une quelconque des revendications précédentes, **caractérisé en ce qu'**il présente une masse molaire moyenne en nombre comprise entre 1 000 et 500 000, notamment entre 5 000 et 100 000 et par exemple entre 10 000 et 50 000.

**10.** Polymère selon l'une quelconque des revendications précédentes, **caractérisé en ce que** le ligand chélatant possède au moins une fonction acide carboxylique et est en particulier choisi parmi l'acide diéthylène triamine penta-acétique (DTPA), l'acide tétraazacyclododacane tétra-acétique (DOTA) et l'acide tétraazacyclotétradécane tétra-acétique (TETA).

**11.** Polymère selon l'une quelconque des revendications précédentes, **caractérisé en ce que** le taux de substitution de l'agent chélatant varie de 0,01 à 40 %, notamment de 0,01 à 10 %, par exemple inférieur à 10 % lorsque le au moins un groupe carbonyle se trouve dans la chaîne principale polymérique et de 0,01 à 40 %, par exemple inférieur à 30 % lorsque le au moins un groupe carbonyle se situe en dehors de la chaîne principale polymérique.

**12.** Procédé de préparation d'un polymère tel que défini selon l'une quelconque des revendications précédentes, **caractérisé en ce qu'**il comprenant (i) au moins une étape d'activation d'un polymère hydrophobe comprenant au moins une unité monomère possédant au moins un groupe carbonyle et comprenant au moins un atome d'hydrogène en α dudit au moins un groupe carbonyle, pour former une chaîne polymérique comportant au moins une unité monomère portant un carbanion par l'élimination du proton situé sur le carbone en α dudit au moins un groupe carbonyle, (ii) au moins une étape de greffage sur ledit polymère comportant au moins une unité monomère portant un carbanion avec un ligand chélatant d'un ion paramagnétique, et (iii) au moins une étape de complexation de l'ion paramagnétique avec l'agent chélatant, par exemple par solubilisation du polymère obtenu à l'étape précédente dans un solvant comprenant au moins un ion paramagnétique.

**13.** Appareil médical, notamment implantable, détectable en imagerie par résonance magnétique, **caractérisé en ce qu'**il comprend un polymère tel que défini selon l'une quelconque des revendications 1 à 11, dans sa masse et/ou à titre de revêtement et/ou à titre de marquage.

**14.** Procédé de préparation d'un dispositif médical notamment implantable détectable en imagerie par résonance magnétique, **caractérisé en ce qu'**il comprend au moins une étape de revêtement par un polymère tel que défini selon l'une quelconque des revendications 1 à 11, notamment par trempage ou par pulvérisation, dans une solution

comprenant ledit polymère.

**15.** Procédé de marquage d'un dispositif médical, **caractérisé en ce qu'**il comprend au moins une étape de dépôt, notamment sur une zone ciblée du dispositif médical, sur la surface du matériau, d'un polymère tel que défini dans l'une quelconque des revendications 1 à 11.

## Patentansprüche

**1.** Hydrophobes Polymer, welches insbesondere zur Herstellung und/oder Beschichtung von medizinischen Geräten, insbesondere implantierbaren Geräten bzw. Instrumenten, die in der Magnetresonanzbildgebung sichtbar sind, geeignet ist, **dadurch gekennzeichnet, dass** es mindestens eine Monomereinheit umfasst, worauf ein komplexierender Ligand eines paramagnetischen Ions gepfropft ist, der mit diesem paramagnetischen Ion komplexiert ist, wobei die Monomereinheit mindestens eine Carbonylgruppe aufweist, die Monomereinheit vor der Pfropfung mindestens ein Wasserstoffatom an der $\alpha$-Position der mindestens einen Carbonylgruppe aufweist und die Pfropfung des komplexierenden Liganden bei dem mindestens einen Wasserstoffatom an der $\alpha$-Position der mindestens einen Carbonylgruppe erfolgt.

**2.** Polymer nach dem vorhergehenden Anspruch, **dadurch gekennzeichnet, dass** die Monomereinheit mit mindestens einer Carbonylgruppe, die vor der Pfropfung ein Wasserstoffatom an der $\alpha$-Position der mindestens einen Carbonylgruppe aufweist, 2 bis 12 Kohlenstoffatome, insbesondere 2 bis 7 Kohlenstoffatome, aufweist.

**3.** Polymer nach Anspruch 1 oder 2, **dadurch gekennzeichnet, dass** sich die Monomereinheit mit mindestens einer Carbonylgruppe, die vor der Pfropfung mindestens ein Wasserstoffatom an der $\alpha$-Position der mindestens einen Carbonylgruppe aufweist, außerhalb der Polymerhauptkette befindet oder alternativ sich die mindestens eine Carbonylgruppe in der Polymerhauptkette befindet.

**4.** Polymer nach einem der vorhergehenden Ansprüche, **dadurch gekennzeichnet, dass** es sich um ein Polyacrylat handelt, welches insgesamt oder teilweise aus identischen oder verschiedenen Monomereinheiten besteht, wobei jede der Einheiten die folgende Formel (I) hat:

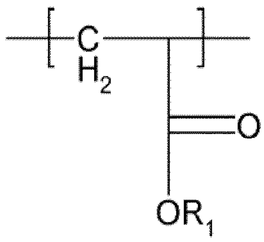

(I)

wobei $R_1$ eine $(C_1\text{-}C_{12})$-Alkylgruppe oder eine $(C_1\text{-}C_8)$-Cycloalkylgruppe, die gegebenenfalls durch eine $(C_1\text{-}C_4)$-Alkylgruppe substituiert ist, darstellt, wobei die Monomereinheit insbesondere von Monomeren abgeleitet sein kann, die aus Butylacrylat, 2-Ethylhexylacrylat, Methylacrylat und Ethylacrylat ausgewählt sind.

**5.** Polymer nach einem der vorhergehenden Ansprüche, **dadurch gekennzeichnet, dass** es von einem Polyester abgeleitet ist, der insgesamt oder teilweise aus identischen oder verschiedenen Monomereinheiten besteht, wobei die Einheiten die folgende Formel (II) aufweisen:

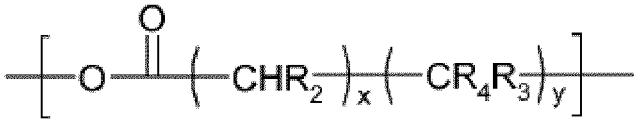

(II)

worin:

$R_2$, $R_3$ und $R_4$ unabhängig ein Wasserstoffatom, eine ($C_1$-$C_{12}$)-Alkylgruppe oder eine ($C_1$-$C_8$)-Cycloalkylgruppe, die gegebenenfalls mit einer ($C_1$-$C_{12}$)-Alkylgruppe substituiert ist, darstellen,
x eine ganze Zahl zwischen 0 und 12 ist, zum Beispiel zwischen 0 und 6, und
y eine ganze Zahl zwischen 0 und 8, zum Beispiel zwischen 0 und 6, ist, wobei x und y nicht gleichzeitig 0 sind.

**6.** Polymer nach dem vorhergehenden Anspruch, **dadurch gekennzeichnet, dass** es durch Polymerisation mindestens eines Monomers erhalten wird, welches ausgewählt ist aus: Hydroxybuttersäure, Hydroxyvaleriansäure, Hydroxyhexansäure und Hydroxyoctansäure; δ-Valerolacton, γ-Butyrolacton; ε-Decalacton; Pivalolacton; Diethylpropriolacton; Glycolsäure; p-Dioxanon (1,4-Dioxan-2-on); 1,4-Dioxepan-2-on; 1,4-Dioxepan-5-on; 1,5-Dioxepan-2-on; 6,6-Dimethyl-1; 4-Dioxan-2-on; 2,5-Diketomorpholin; 3-Methyl-1,4-dioxan-2,5-dion; 3,3-Diethyl-1,4-dioxan-2,5-dion; 6,6-dimethyldioxepan-2-on und Mischungen davon.

**7.** Polymer nach einem der vorhergehenden Ansprüche, **dadurch gekennzeichnet, dass** es aus Poly(ε-caprolacton) erhalten wird.

**8.** Polymer nach einem der vorhergehenden Ansprüche, **dadurch gekennzeichnet, dass** es sich um ein Homopolymer oder ein Copolymer handelt, insbesondere ein statistisches Copolymer, ein Blockcopolymer oder ein Pfropfcopolymer, kammartig oder alternierend.

**9.** Polymer nach einem der vorhergehenden Ansprüche, **dadurch gekennzeichnet, dass** es ein Zahlenmittel der Molmasse zwischen 1000 und 500000 aufweist, insbesondere zwischen 5000 und 100000, und beispielsweise zwischen 10000 und 50000.

**10.** Polymer nach einem der vorhergehenden Ansprüche, **dadurch gekennzeichnet, dass** der komplexierende Ligand mindestens eine Carbonsäurefunktion aufweist und insbesondere aus Diethylentriaminpentaessigsäure (DTPA), Tetraazacyclododecantetraessigsäure (DOTA) und Tetraazacyclotetradecantetraessigsäure (TETA) ausgewählt ist.

**11.** Polymer nach einem der vorhergehenden Ansprüche, **dadurch gekennzeichnet, dass** der Substitutionsgrad des Komplexierungsmittels zwischen 0,01 bis 40% variiert, insbesondere von 0,01 bis 10%, beispielsweise weniger als 10%, wenn sich die mindestens eine Carbonylgruppe in der Polymerhauptkette befindet, und von 0,01 bis 40%, zum Beispiel weniger als 30%, wenn sich die mindestens eine Carbonylgruppe außerhalb der Polymerhauptkette befindet.

**12.** Verfahren zur Herstellung eines Polymers nach einem der vorhergehenden Ansprüche, **dadurch gekennzeichnet, dass** es umfasst:

(i) mindestens einen Aktivierungsschritt eines hydrophoben Polymers, welches mindestens eine Monomereinheit umfasst, die mindestens eine Carbonylgruppe aufweist und mindestens ein Wasserstoffatom an der α-Position der mindestens einen Carbonylgruppe aufweist, um eine Polymerkette zu bilden, die mindestens eine Monomereinheit enthält, die durch Entfernung des Protons an dem Kohlenstoff an der α-Position der mindestens einen Carbonylgruppe ein Carbanion enthält,
(ii) mindestens einen Schritt des Pfropfens mit einem komplexierenden Liganden eines paramagnetischen Ions auf das Polymer, welches mindestens eine Monomereinheit umfasst, die ein Carbanion aufweist, und
(iii) mindestens einen Komplexierungsschritt des paramagnetischen Ions mit dem Komplexbildner, beispielsweise durch Solubilisieren des in dem vorhergehenden Schritt erhaltenen Polymers in einem Lösungsmittel, welches mindestens ein paramagnetisches Ion enthält.

**13.** Medizinisches Gerät, insbesondere zur Implantation, welches durch Magnetresonanzbildgebung detektierbar ist, **dadurch gekennzeichnet, dass** es ein Polymer wie in einem der Ansprüche 1 bis 11 definiert in seiner Masse und/oder als Beschichtung und/oder als Markierung umfasst.

**14.** Verfahren zur Herstellung eines medizinischen Geräts/Instruments, insbesondere zur Implantation, das durch Magnetresonanzbildgebung detektierbar ist, **dadurch gekennzeichnet, dass** es mindestens einen Schritt der Beschichtung mit einem wie in einem der Ansprüche 1 bis 11 definierten Polymer umfasst, insbesondere durch Tränken in einer Lösung, welche das Polymer enthält, oder durch Versprühen einer Lösung, welche das Polymer enthält.

**15.** Verfahren zur Herstellung eines medizinischen Geräts/Instruments, **dadurch gekennzeichnet, dass** es mindestens

einen Schritt der Ablagerung des Polymers nach einem der Ansprüche 1 bis 11, insbesondere auf einer Zielzone des medizinischen Geräts/Instruments, auf der Oberfläche des Materials umfasst.

## Claims

1. A hydrophobic polymer, notably useful for making and/or coating medical devices, notably implantable, visible in magnetic resonance imaging, **characterized in that** it comprises at least one monomer unit on which a chelating ligand of a paramagnetic ion complexed with said paramagnetic ion is grafted, said monomer unit possessing at least one carbonyl group, said monomer unit comprising, prior to grafting, at least one hydrogen atom in the $\alpha$ position of said at least one carbonyl group and said grafting of the chelating ligand taking place at the level of said at least one hydrogen atom in the $\alpha$ position of said at least one carbonyl group.

2. The polymer as claimed in the preceding claim, **characterized in that** the monomer unit possessing at least one carbonyl group and comprising, prior to grafting, a hydrogen atom in the $\alpha$ position of said at least one carbonyl group, comprises from 2 to 12 carbon atoms, in particular from 2 to 7 carbon atoms.

3. The polymer as claimed in one of claims 1 or 2, **characterized in that** the monomer unit possessing at least one carbonyl group and comprising, prior to grafting, at least one hydrogen atom in the $\alpha$ position of said at least one carbonyl group is located outside the main polymer chain or alternatively said at least one carbonyl group is located in the main polymer chain.

4. The polymer as claimed in any one of the preceding claims, **characterized in that** it is a polyacrylate consisting wholly or partly of identical or different monomer units, each of said units having the following formula (I)

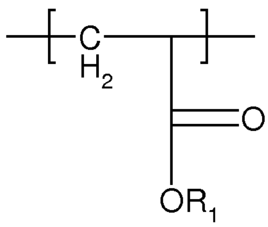
(I)

in which $R_1$ represents a $(C_1\text{-}C_{12})$alkyl group or a $(C_1\text{-}C_8)$cycloalkyl group optionally substituted with a $(C_1\text{-}C_4)$alkyl group, and said monomeric unit can notably be derived from monomers selected from butyl acrylate, 2-ethylhexyl acrylate, methyl acrylate and ethyl acrylate.

5. The polymer as claimed in any one of the preceding claims, **characterized in that** it is derived from a polyester consisting wholly or partly of identical or different monomer units, each of said units having the following formula (II):

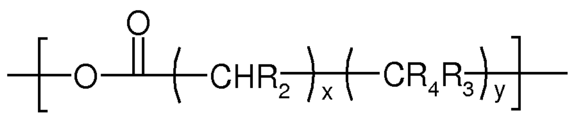
(II)

in which:

$R_2$, $R_3$ and $R_4$ independently represent a hydrogen atom, a $(C_1\text{-}C_{12})$ alkyl group or a $(C_1\text{-}C_8)$ cycloalkyl group optionally substituted with a $(C_1\text{-}C_{12})$ alkyl group,
x represents an integer between 0 and 12, for example between 0 and 6, and
y represents an integer between 0 and 8, for example between 0 and 6, it being understood that x and y are not zero simultaneously.

6. The polymer as claimed in the preceding claim, **characterized in that** it is derived from the polymerization of at least one monomer selected from hydroxybutyric acid, hydroxyvaleric acid, hydroxyhexanoic acid and hydroxyoctanoic acid; $\delta$-valerolactone; $\gamma$-butyrolactone; $\varepsilon$-decalactone; pivalolactone; diethylpropiolactone; glycolic acid; p-dioxanone (1,4-dioxan-2-one); 1,4-dioxepan-2-one; 1,4-dioxepan-5-one; 1,5-dioxepan-2-one; 6,6-dimethyl-1; 4-dioxan-2-one; 2,5-diketomorpholine; 3-methyl- 1,4-dioxane-2,5-dione; 3,3-diethyl- 1,4-dioxan-2,5-dione; 6,6-dimethyl-dioxepan-2-one and mixtures thereof.

**7.** The polymer as claimed in any one of the preceding claims, **characterized in that** it is derived from poly(ε-caprolactone).

**8.** The polymer as claimed in any one of the preceding claims, **characterized in that** it is a homopolymer, or a copolymer, notably random or block, grafted, in comb fashion or alternating.

**9.** The polymer as claimed in any one of the preceding claims, **characterized in that** it has a number-average molecular weight between 1000 and 500 000, notably between 5000 and 100 000 and for example between 10 000 and 50 000.

**10.** The polymer as claimed in any one of the preceding claims, **characterized in that** the chelating ligand has at least one carboxylic acid function and is in particular selected from diethylene triamine pentaacetic acid (DTPA), tetraazacyclododacane tetraacetic acid (DOTA) and tetraazacyclotetradecane tetraacetic acid (TETA).

**11.** The polymer as claimed in any one of the preceding claims, **characterized in that** the degree of substitution of the chelating agent varies from 0.01 to 40%, notably from 0.01 to 10%, for example is less than 10% when at least one carbonyl group is located in the main polymer chain and from 0.01 to 40%, for example less than 30% when at least one carbonyl group is located outside the main polymer chain.

**12.** A method of preparing a polymer as defined in any one of the preceding claims, **characterized in that** it comprises (i) at least one step of activation of a hydrophobic polymer comprising at least one monomer unit possessing at least one carbonyl group and comprising at least one hydrogen atom in the α position of said at least one carbonyl group, to form a polymer chain having at least one monomer unit bearing a carbanion by elimination of the proton located on the carbon in the α position of said at least one carbonyl group, (ii) at least one step of grafting on said polymer having at least one monomer unit bearing a carbanion with a chelating ligand of a paramagnetic ion, and (iii) at least one step of complexation of the paramagnetic ion with the chelating agent, for example by dissolution of the polymer obtained in the preceding step in a solvent comprising at least one paramagnetic ion.

**13.** A medical device, notably implantable, detectable in magnetic resonance imaging, **characterized in that** it comprises a polymer as defined in any one of claims 1 to 11, in its bulk and/or as a coating and/or as a marker.

**14.** A method of preparing a medical device, notably implantable, detectable in magnetic resonance imaging, **characterized in that** it comprises at least one step of coating with a polymer as defined in any one of claims 1 to 11, notably by dipping or by spraying, in a solution comprising said polymer.

**15.** A method of marking of a medical device, **characterized in that** it comprises at least one step of deposition, notably on a target zone of the medical device, on the surface of the material, of a polymer as defined in any one of claims 1 to 11.

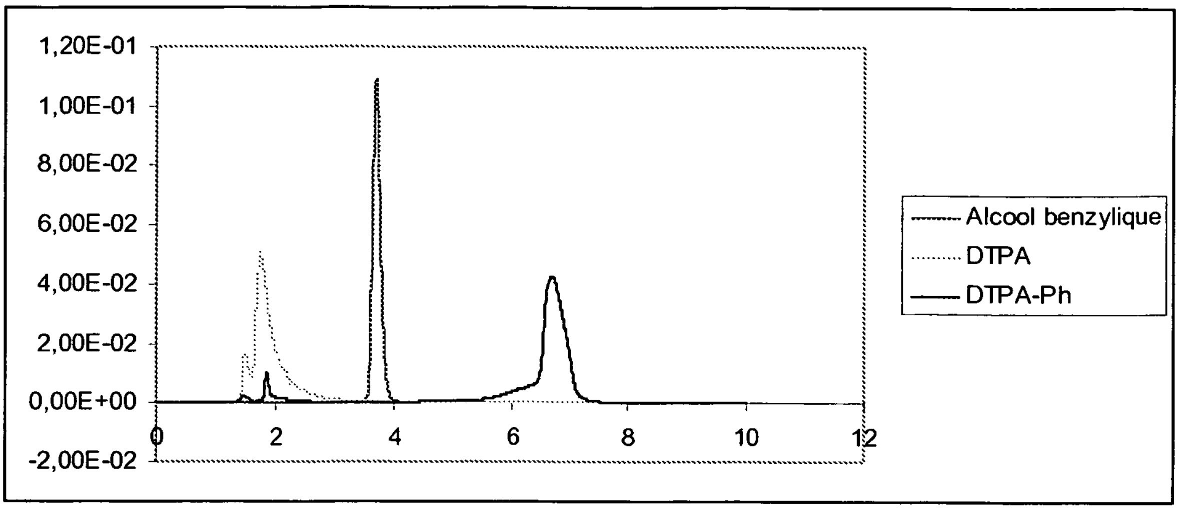


**FIGURE 1**

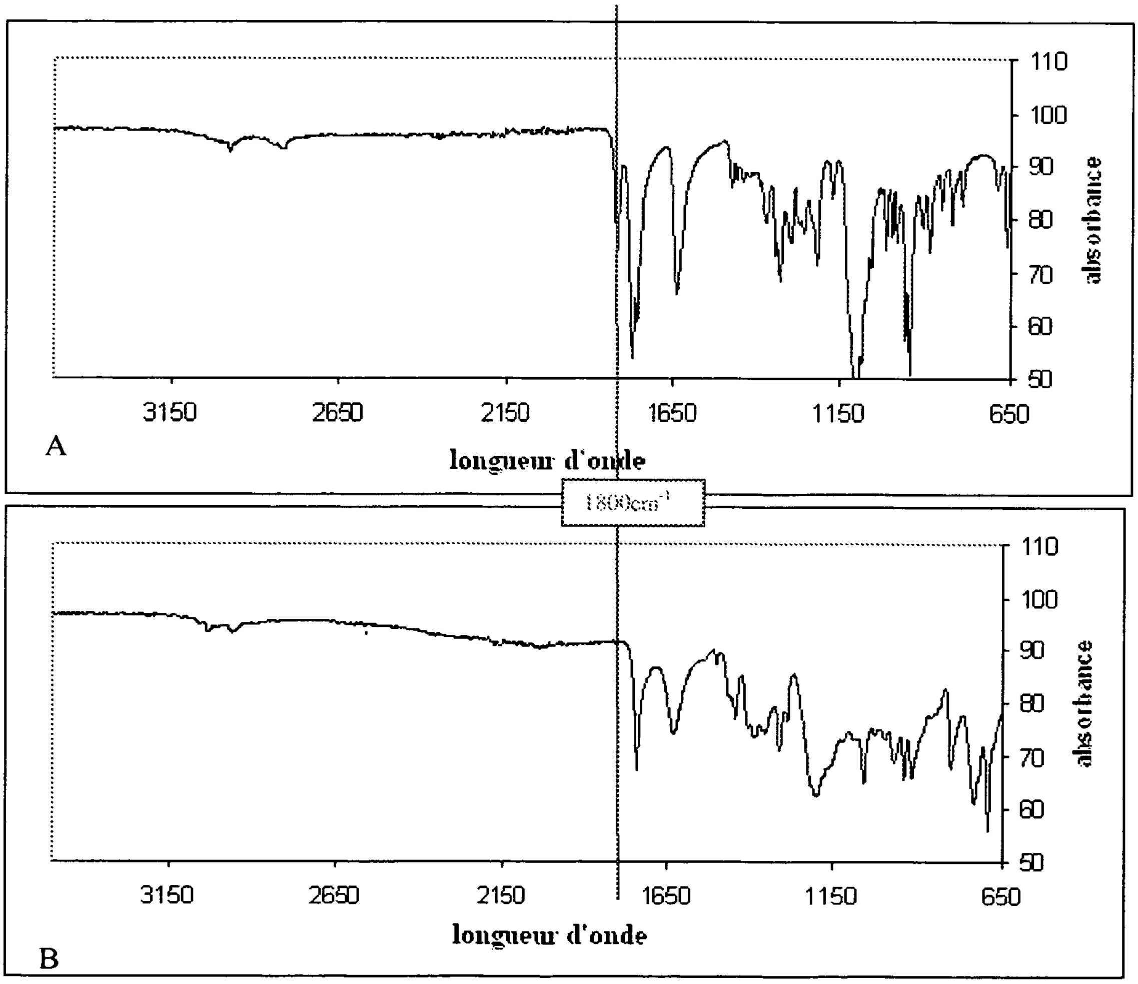


**FIGURE 2**

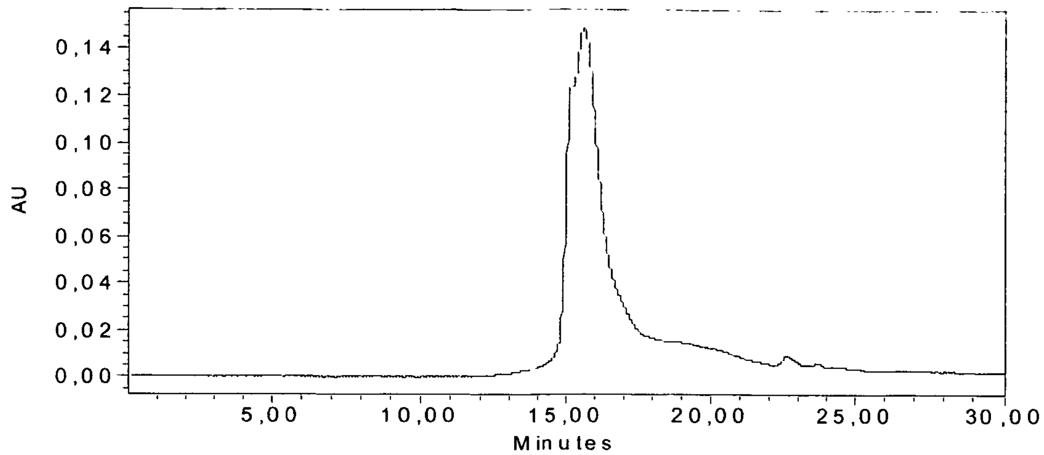


**FIGURE 3**

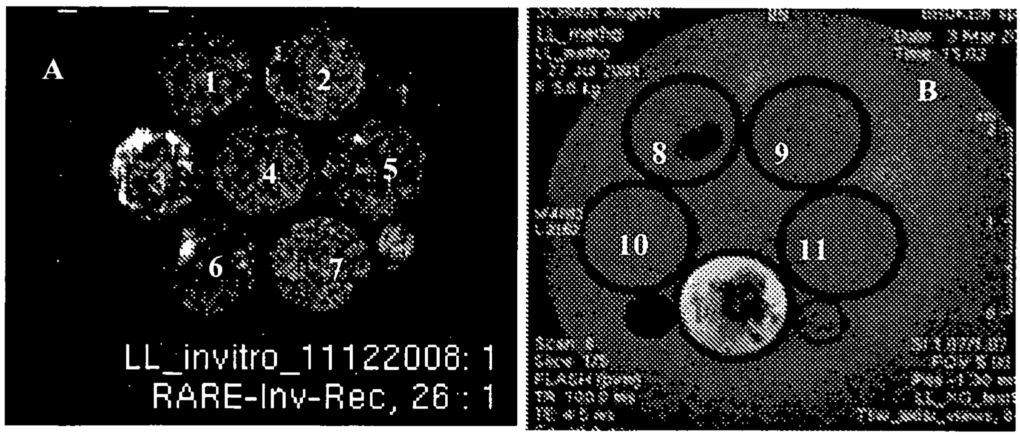


Fig. 4-A

Fig. 4-B

**FIGURE 4**

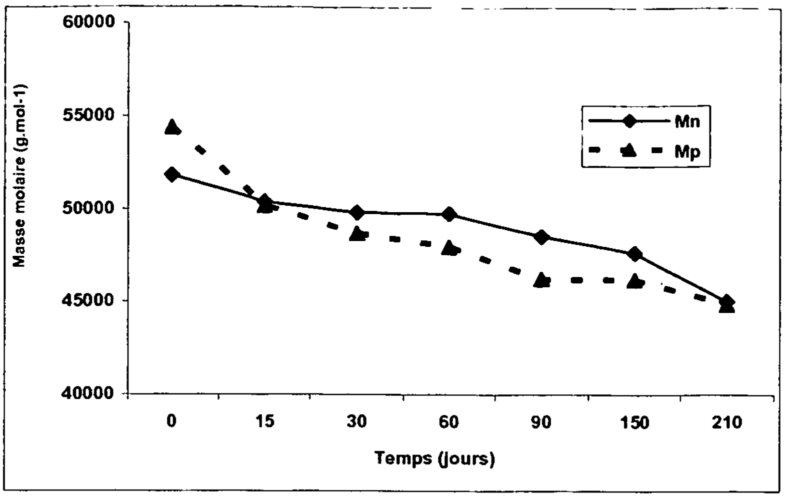
60000
55000
50000
45000
40000
Masse molaire (g.mol-1)
0
15
30
60
90
150
210
Temps (jours)
Mn
Mp

FIGURE 5

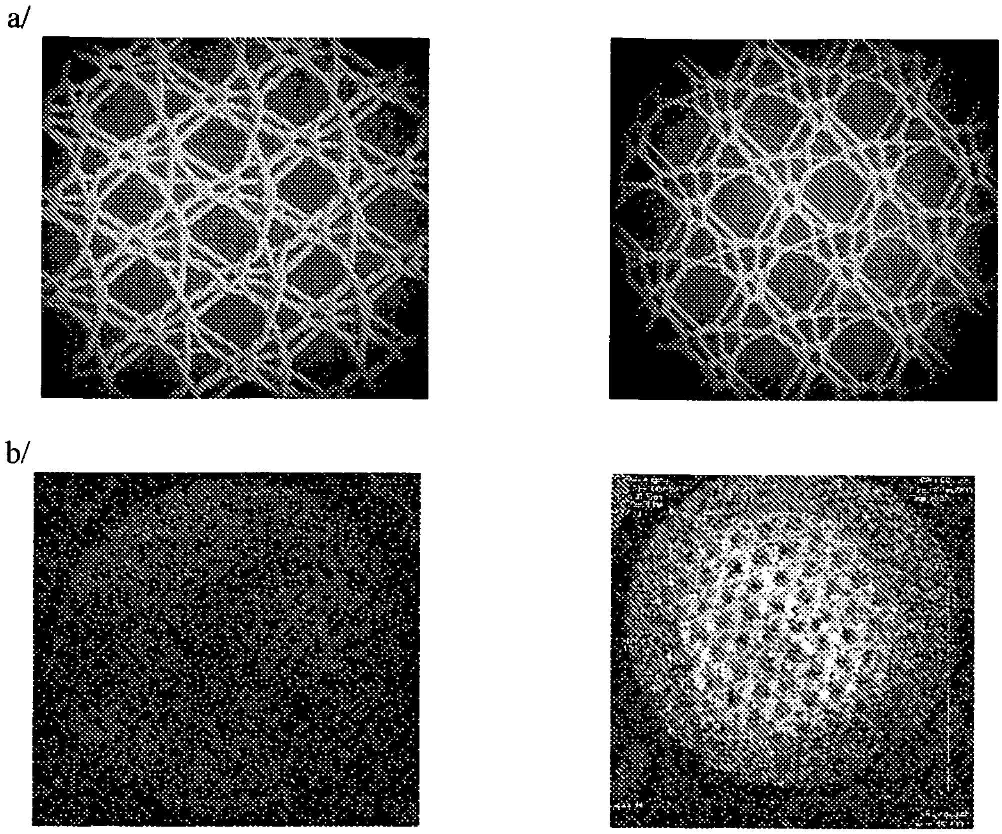


FIGURE 6

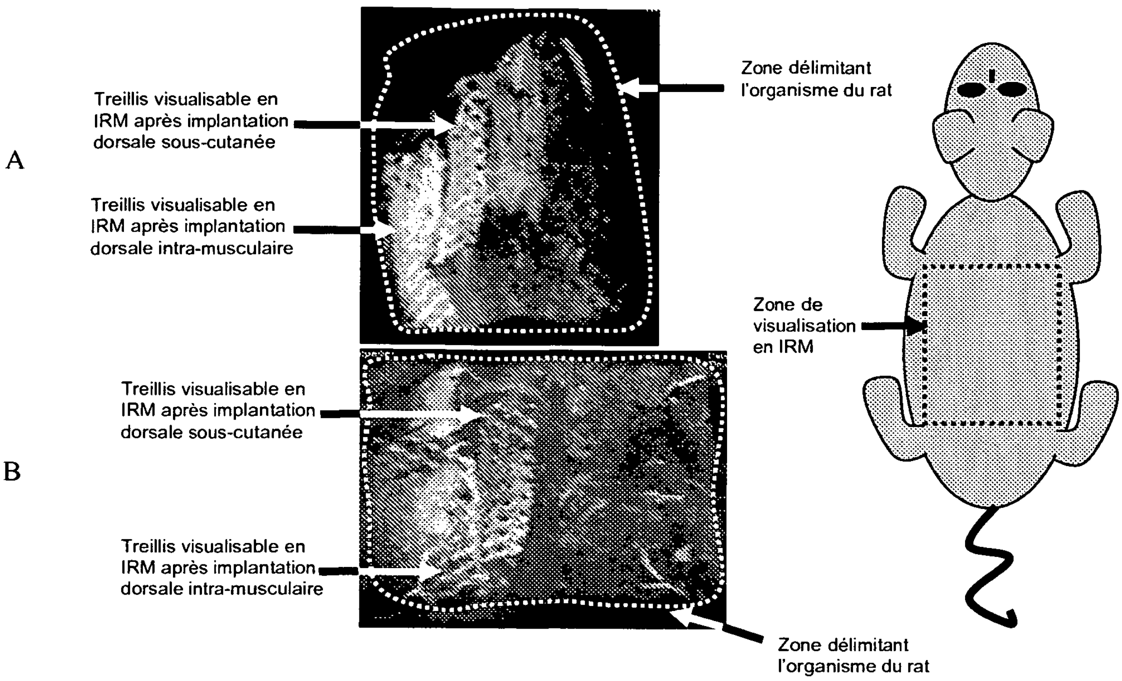
A
Treillis visualisable en IRM après implantation dorsale sous-cutanée
Treillis visualisable en IRM après implantation dorsale intra-musculaire
Zone délimitant l'organisme du rat
B
Treillis visualisable en IRM après implantation dorsale sous-cutanée
Treillis visualisable en IRM après implantation dorsale intra-musculaire
Zone délimitant l'organisme du rat
Zone de visualisation en IRM

FIGURE 7

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

### Documents brevets cités dans la description


- WO 03045457 A
- US 4822594 A
- WO 9960920 A


### Littérature non-brevet citée dans la description


- **S. PONSART et al.** A Novel Route to Poly(ε-caprolactone) Based Copolymers Via Anionic Derivativation. *Biomacromolecules,* 2000, vol. 1, 275-281